# EUROPÄISCHE PATENTSCHRIFT

(11) **EP 1 088 813 B1**
(45) Veröffentlichungstag und Bekanntmachung des Hinweises auf die Patenterteilung: **03.12.2003**
(21) Anmeldenummer: 00119683.1
(22) Anmeldetag: 08.09.2000
(51) Int. Cl.: C07C 271/12, C07C 271/28, C07C 269/02, C08F 2/50

(54) **Urethangruppen enthaltende Photoinitiatoren zur kationischen Härtung**
Urethane group containing photoinitiators for cationic curing
Photoinitiateurs contenant des groupes d'uréthane pour le durcissement cationique

(30) Priorität: 21.09.1999 DE 19945251
(43) Veröffentlichungstag der Anmeldung: 04.04.2001
(73) Patentinhaber: Goldschmidt AG, 45127 Essen (DE)
(72) Erfinder: Oestreich, Sascha, Dr., 45279 Essen (DE); Volkmer, Stefanie, 45289 Essen (DE); Weier, Andreas, Dr., 45289 Essen (DE)

(56) Entgegenhaltungen:
- EP-A- 1 020 479
- WO-A-93/13110
- US-A- 5 073 643
- US-A- 5 079 378

## Beschreibung

Die vorliegende Erfindung betrifft Urethangruppen enthaltende Iodoniumsalze mit verringerter Kristallisationsneigung, ein Verfahren zu deren Herstellung sowie deren Verwendung zur Strahlenhärtung kationisch härtender Massen.

Die kationische Photopolymerisation ist ein schneller, effizienter und umweltschonender Weg, um kationisch polymerisierbare Monomere zu härten. Besonders effiziente Photoinitiatoren stellen Diaryliodonium- (I) und Triarylsulfoniumsalze (II) dar.

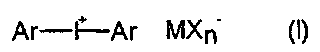

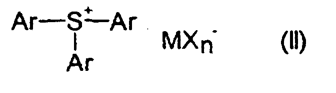

MX⁻ₙ = BF₄⁻, PF₆⁻, AsF₆⁻, SbF₆⁻

Insbesondere Diaryliodoniumsalze (I) sind aus der Patentliteratur bekannt (DE-A-25 18 639, US-A-4 279 717, EP-A-0 334 056, US-A-5 079 378, WO-A-93/13110 EP-B-0 618 919) und werden als Photoinitiatoren zur Polymerisation von kationisch polymerisierbaren Substanzen verwendet. Die kationisch polymerisierbaren Substanzen können aber unpolar bis wenig polar sein, besonders wenn die polymerisierbaren Gruppen in Organopolysiloxanen enthalten sind. Es wird daher beim Zusatz dieser Photoinitiatoren sehr häufig beobachtet, daß, abhängig von der Struktur der Zubereitung, die Mischbarkeit und Löslichkeit der Photoinitiatoren begrenzt ist. Daher werden die Arylreste solcher Oniumsalze oft mit Alkylketten substituiert, um die Löslichkeit in Organopolysiloxanen zu erhöhen (US-A-4 310 469 und US-A-4 374 066).

Bei Hydroxylgruppen tragenden Iodoniumsalzen, wie sie in US-A-5 073 643 beschrieben werden, wird die schlechte Löslichkeit in unpolaren Medien auf die hohe Kristallisationsneigung zurückgeführt. Das besondere Komplexbildungsverhalten der Hydroxylgruppen tragenden Iodoniumsalze der allgemeinen Formel (III) führt zu einer starken Kristallisationsneigung der Verbindungen (A. Kunze, U. Müller, K. Tittes, J.P. Fouassier, F. Morlet-Savary, J. Photochemistry and Photobiology A: Chemistry, 110, 115-122 (1997)):

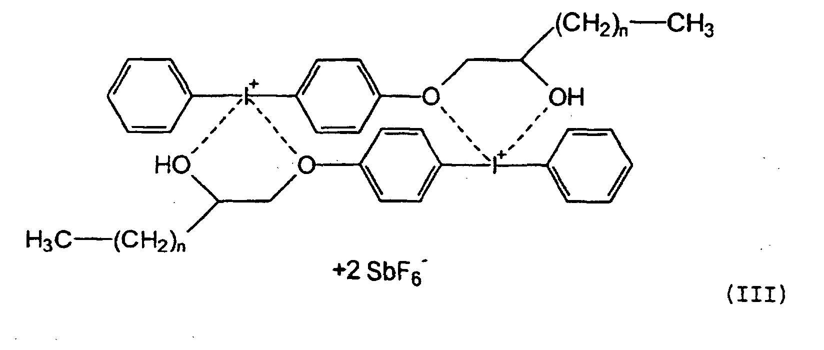

Die beiden Sauerstoffatome des Moleküls wirken als Liganden für ein zweites Iodoniumion. Durch dieses Aggregationsverhalten wird die Ausbildung von Kristallen gefördert.

Bei der Herstellung dieser Salze ist die starke Kristallisationsneigung durchaus erwünscht, da dadurch die Verbindungen durch einfache Umkristallisation in hoher Reinheit als Pulver gewonnen werden können. Auf diese Weise sind sie einfach und kostengünstig herstellbar. Solche Iodoniumsalze sind zum Beispiel unter dem Namen CD-1012 von Sartomer kommerziell erhältlich.

Die hohe Kristallisationsneigung wirkt sich allerdings negativ aus, wenn die Iodoniumsalze in unpolaren Medien, wie Organopolysiloxanen, gelöst werden sollen. Hier sind sie entweder unlöslich oder es bildet sich schon nach kurzer Zeit ein fester Niederschlag.

Aufgrund ihrer Inhomogenität härten solche Beschichtungen bei UV-Bestrahlung nur schlecht aus. Es ist aber auch möglich, daß durch die Inhomogenität schon während der Applikation einer dünnen Schicht auf einem Substrat massive Oberflächenstörungen (Krater, Runzeln, Stippen etc.) auftreten.

Der vorliegenden Erfindung liegt die Aufgabe zugrunde, Hydroxylgruppen tragende Iodoniumsalze auf eine besonders kostengünstige, einfache Art so zu modifizieren, daß die Kristallisationsneigung stark herabgesetzt wird, eine gute Verträglichkeit insbesondere mit Epoxygruppen aufweisenden Organopolysiloxanen hergestellt wird und die entstehenden Verbindungen hydrolysestabil sind, so daß bei der Lagerung keine Hydroxylgruppen tragenden Iodoniumsalze zurückgebildet werden können.

Die vorgenannte Aufgabe wird gelöst durch Iodoniumsalze der allgemeinen Formel (IV)

[R¹-I-R²]⁺ X⁻ (IV)

wobei
- I: Iod ist,
- X⁻: ein Anion eines komplexen Metallsalzes und/oder einer starken Säure ist,
- R¹: der Rest 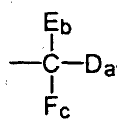 ist, wobei
- C: ein einwertiger aromatischer Kohlenwasserstoffrest mit 6 bis 14 Kohlenstoffatomen je Rest oder ein einwertiger, mindestens ein Sauerstoff- und/oder Schwefelatom enthalten-der aromatischer Kohlenwasserstoffrest mit 5 bis 15 Ringatomen je Rest ist,
- a: 1, 2 oder 3,
- b: 0, 1 oder 2,
- c: 0, 1 oder 2 sind,
- D, E und F: jeweils Substituenten von C sind, wobei
- D: ein Rest der Formel 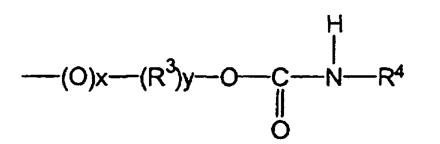 ist, wobei
- x: 0 oder 1,
- y: 0 oder 1 sind,
- R³: ein linearer oder verzweigter zweiwertiger Kohlenwasserstoffrest mit 1 bis 40 Kohlenstoffatomen je Rest, der gegebenenfalls durch mindestens ein Sauerstoffatom und/oder mindestens ein Schwefelatom und/oder mindestens eine Carboxylgruppe unterbrochen sein kann, ist,
- R⁴: ein einwertiger linearer, verzweigter und/oder cyclischer Alkyl-, Aryl-, Haloalkyl- und/oder Haloarylradikalrest mit 1 bis 40 Kohlenstoffatomen ist, der gegebenenfalls durch mindestens ein Sauerstoffatom und/oder mindestens ein Schwefelatom und/oder mindestens eine Urethan- und/oder Estergruppe unterbrochen sein kann, und der gegebenenfalls hydrophobierende Substituenten und/oder mindestens eine Isocyanatgruppe enthalten kann,
- E: ein Rest der Formel

-O-R⁵
- F: ein Rest der Formel

-R⁶
- R²: ein Rest der Formel 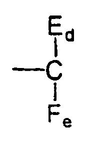 ist, wobei
- R⁵: ein einwertiger Kohlenwasserstoffrest mit 1 bis 18 Kohlen stoffatomen je Rest ist, der gegebenenfalls durch mindestens ein Sauerstoffatom unterbrochen sein kann,
- R⁶: ein einwertiger Kohlenwasserstoffrest mit 1 bis 18 Kohlenstoffatomen je Rest ist, der gegebenenfalls durch mindestens ein Sauerstoffatom unterbrochen sein kann,
- d: 0, 1 oder 2 und
- e: 0, 1 oder 2 sind.

Überraschenderweise wurde gefunden, daß durch die Bildung eines Urethans aus Hydroxylgruppen enthaltenden Iodoniumsalzen die Kristallisationsneigung erheblich herabgesetzt, die Verträglichkeit mit Epoxygruppen aufweisenden Organopolysiloxanen wesentlich verbessert und die Hydrolysestabilität gegenüber Si-O-C-Bindungen enthaltenden Iodoniumsalzen (siehe Deutsche Patentanmeldung 19901531.7) erheblich erhöht werden kann.

Bevorzugte Beispiele für aromatische Kohlenwasserstoffreste C sind der Phenyl-, Naphthyl- und Anthrylrest.

Bevorzugte Beispiele für mindestens ein Sauerstoff- und/oder Schwefelatom enthaltende aromatische Kohlenwasserstoffreste C sind der 2-Furyl-, 3-Furyl-, 2-Thienyl- und 3-Thienylrest.

Bevorzugte Beispiele für den zweiwertigen Kohlenwasserstoffrest R³, der durch mindestens ein Sauerstoffatom und/oder mindestens ein Schwefelatom und/oder eine Carboxylgruppe unterbrochen sein kann, sind -CH₂-, -CH₂-CH₂-, -CH₂-CH₂-O-CH₂-CH₂-, -CH₂-CH(CH₃)-, -CH₂-CH(CH₂CH₃)-, -CH₂-CH((CH₂)₃CH₃)-, -CH₂-CH((CH₂)₁₁CH₃)-, -CH₂-CH((CH₂)₁₃CH₃)-, -CH₂-CH₂-S-CH₂-CH₂-, -CH₂-O-C(O)-CH₂-.

Bevorzugte Beispiele für R⁴, den einwertigen linearen, verzweigten und/oder cyclischen Alkyl-, Aryl-, Haloalkylund/oder Haloarylradikalrest mit 1 bis 40 Kohlenstoffatomen, der gegebenenfalls durch mindestens ein Sauerstoffatom und/oder mindestens ein Schwefelatom und/oder mindestens eine Urethanund/oder Estergruppe unterbrochen sein kann, und der gegebenenfalls hydrophobierende Substituenten und/oder mindestens eine Isocyanatgruppe enthalten kann, sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest; Hexylreste, wie der n-Hexylrest, wie der Cyclohexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest und Octyldecylreste, wie der n-Octadecylrest; Arylreste, wie der Phenyl-, Naphthyl- und Anthrylrest; Haloalkylreste, wie der Chlormethyl-, Trichlormethyl-, Trifluormethyl-, Pentafluorethyl- und Heptafluorpropylrest; Haloarylreste, wie der Bromphenyl-, Chlorphenyl-, Fluorphenyl-, Difluorphenyl-, Fluor(trifluormethyl)phenyl-, Pentafluorphenyl-, (Trifluormethyl)phenyl-, (Trifluormethylthio)phenyl- und Trifluormethoxyphenylrest.

Die vorgenannten Beispiele für den Rest R⁴ gelten in vollem Umfang auch für die Reste R⁵ und R⁶.

Beispiele für durch mindestens ein Sauerstoffatom und/oder Schwefelatom unterbrochene Kohlenwasserstoffe R⁴, R⁵ und R⁶ sind -CH₂-CH₂-O-CH₃, -CH₂-CH₂O-CH₂CH₃ und -CH₂-CH₂-S-CH₂CH₃.

Beispiele für den durch mindestens eine Urethan- und/oder Estergruppe unterbrochenen Rest R⁴, der gegebenenfalls hydrophobierende Substituenten und/oder mindestens eine Isocyanatgruppe enthalten kann, sind

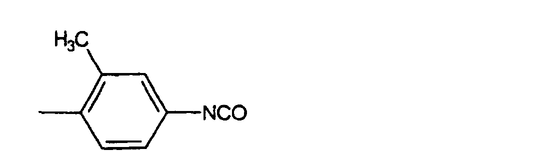

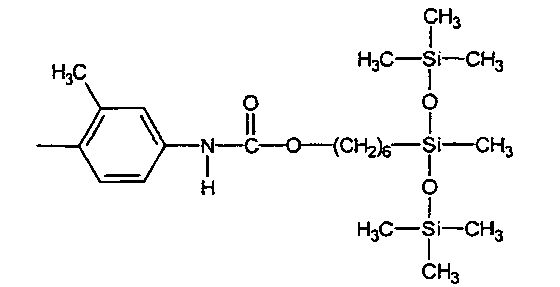

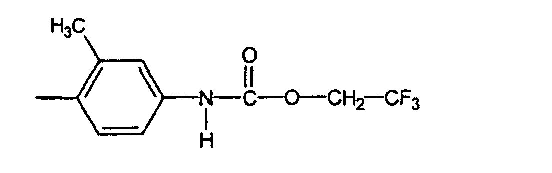

Bevorzugte Beispiele für Reste D sind

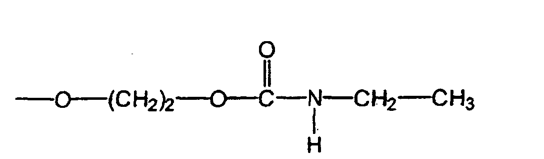

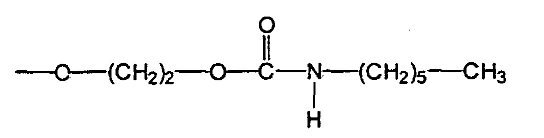

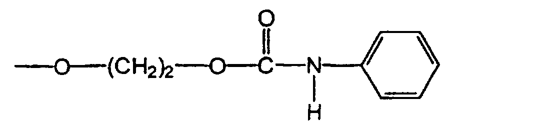

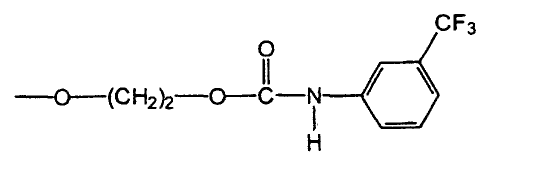

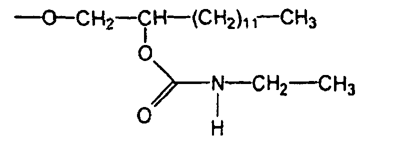

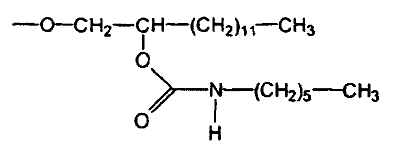

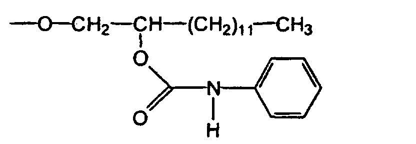

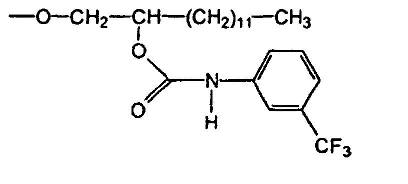

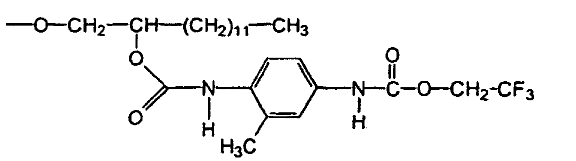

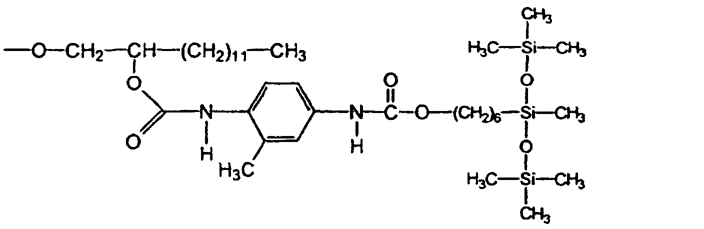

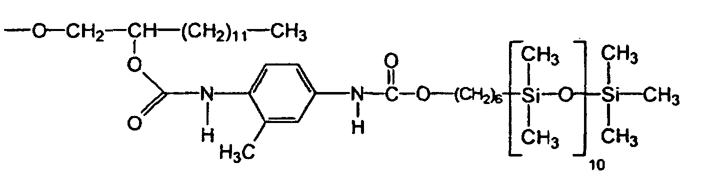

Bevorzugte Beispiele für Reste E sind der Methoxy-, Ethoxy- und der n-Butoxyrest.

Bevorzugte Beispiele für Reste F sind der Methyl-, Ethyl-, Propyl-, 2-Methylpropyl- und der n-Butylrest.

Bevorzugte Beispiele für Reste R¹ sind

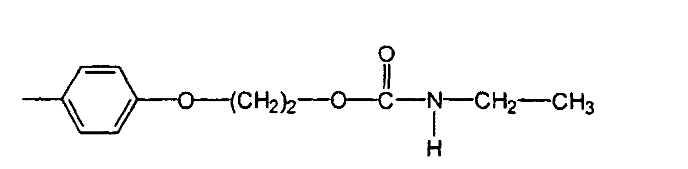

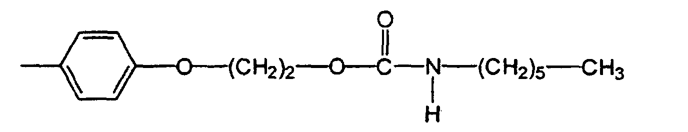

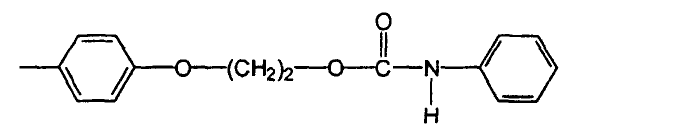

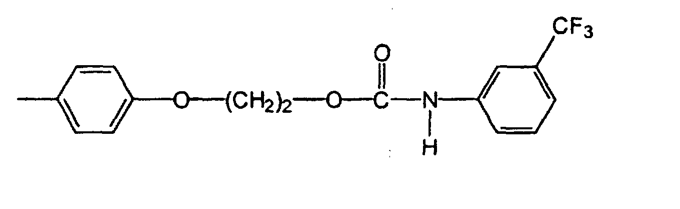

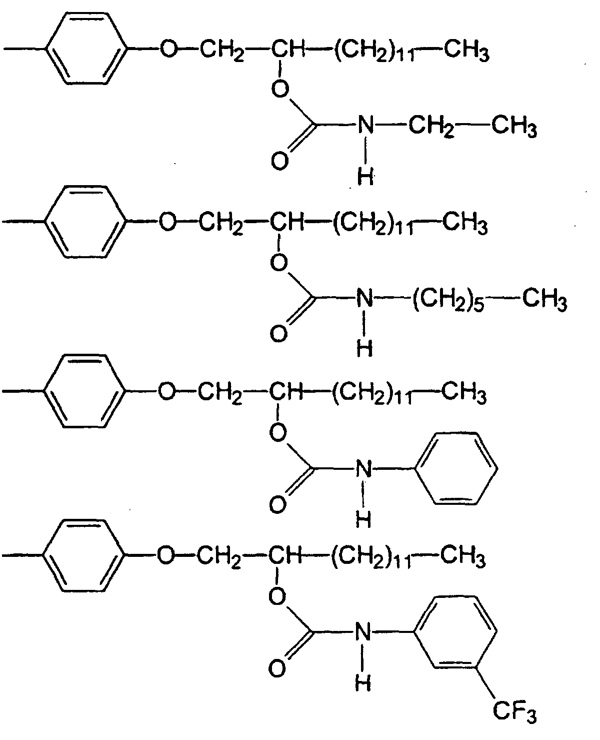

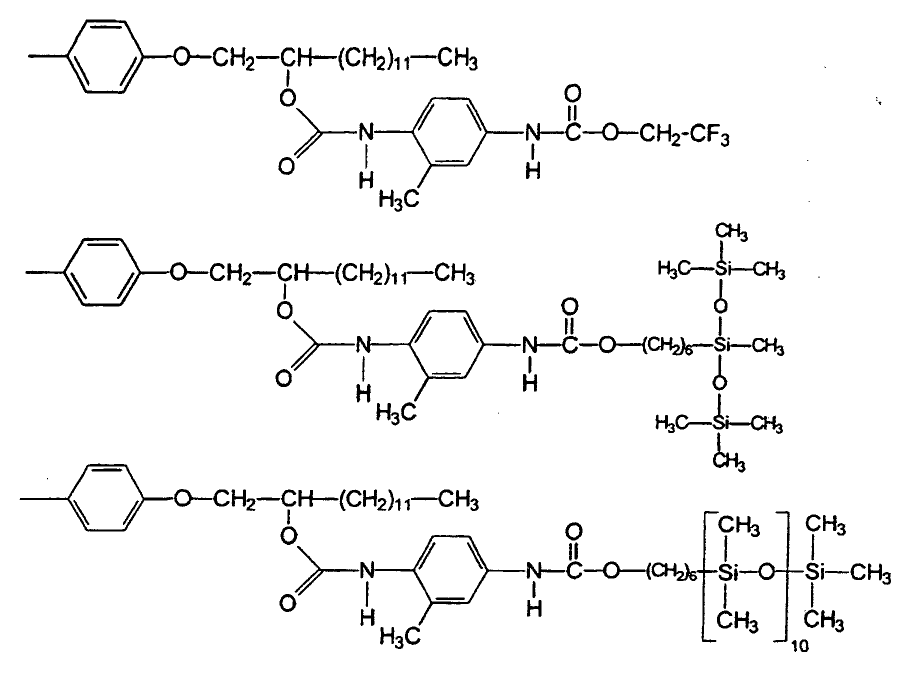

Bevorzugte Beispiele für Reste R² sind der Phenyl-, 4-Methylphenyl-, 3-Methoxyphenyl- und 4-Methoxyphenylrest.

Bevorzugte Beispiele für Anionen X⁻ eines komplexen Metallsalzes und/oder einer starken Säure sind Tosylat, SbF₆⁻, PF₆⁻, BF₄⁻, F₃CSO₃⁻, F₃CCO₂⁻, AsF₆⁻, ClO₄⁻, HSO₄⁻. Starke Säuren im Sinne der vorliegenden Erfindung umfassen insbesondere starke Brönstedt-Säuren.

Bevorzugt sind als Iodoniumsalze mit verminderter Kristallisationsneigung solche der allgemeinen Formel (V) 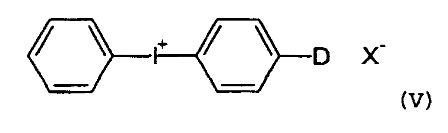 wobei D und X⁻ die bereits angegebene Bedeutung haben.

Besonders bevorzugt sind Iodoniumsalze mit verminderter Kristallisationsneigung der Formel (VI) 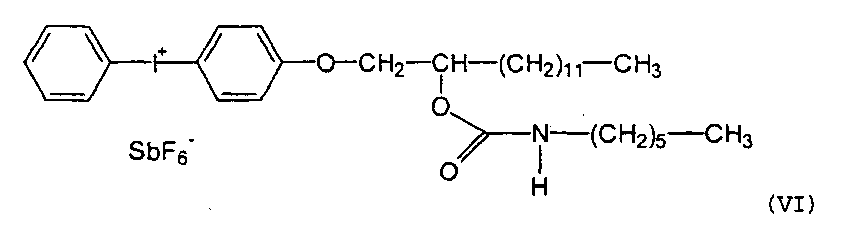 und der Formel (VII)

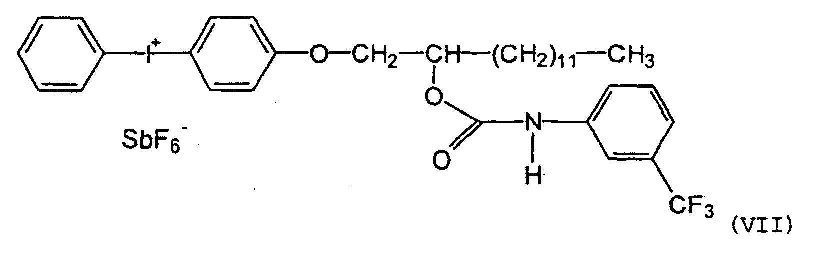

Die Kristallisationsneigung der so modifizierten Iodoniumsalze ist gegenüber dem eingangs erwähnten Stand der Technik wesentlich herabgesetzt. Beispielsweise stellt das Iodoniumsalz (VII) bei Raumtemperatur eine viskose Flüssigkeit dar, während das vergleichbare Hydroxylgruppen enthaltende Iodoniumsalz mit der nachfolgenden Formel (VIII) 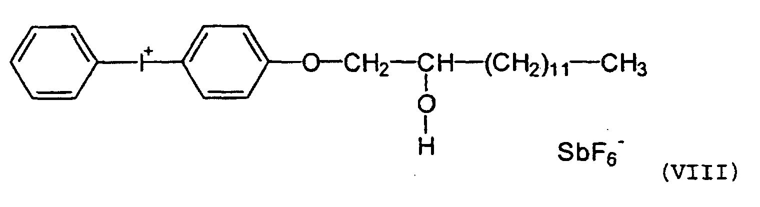 ein Pulver mit einem Schmelzpunkt von 91 °C darstellt, welches unter dem Handelsnamen CD-1012 von Sartomer erhältlich ist.

Ebenso ist die Löslichkeit der erfindungsgemäßen Iodoniumsalze in unpolaren Medien, wie n-Alkanen oder Siloxanen, wesentlich größer als die der vergleichbaren Hydroxylgruppen enthaltenden Iodoniumsalze.

Beispielsweise ist das Iodoniumsalz der allgemeinen Formel (VII) unbegrenzt in Toluol löslich. Dagegen ist das vergleichbare, Hydroxylgruppen enthaltende Iodoniumsalz der allgemeinen Formel (VIII) unlöslich in Toluol und zeigt keine Mischbarkeit mit Epoxygruppen enthaltenden Organopolysiloxanen.

Gegenstand der Erfindung ist weiterhin ein Verfahren zur Herstellung der Iodoniumsalze mit verminderter Kristallisationsneigung. Erfindungsgemäße Iodoniumsalze werden bequemerweise durch Umsetzungen mit Isocyanaten hergestellt.

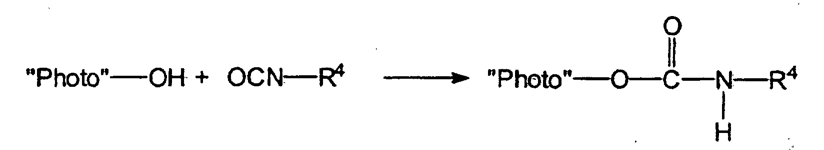

Wobei "Photo" einen zu modifizierenden Photoinitiatorrest bezeichnet.

Das erfindungsgemäße Verfahren ist dadurch gekennzeichnet, daß ein Hydroxylgruppen enthaltendes Iodoniumsalz mit der allgemeinen Formel (IX)

[R¹-I-R²]⁺ X⁻ (IX)

wobei
- I: Iod ist,
- X⁻: ein Anion eines komplexen Metallsalzes und/oder einer starken Säure ist,
- R¹: ein Rest 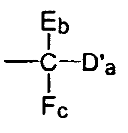 ist, worin
- C: ein einwertiger aromatischer Kohlenwasserstoffrest mit 6 bis 14 Kohlenstoffatomen je Rest oder ein einwertiger, mindestens ein Sauerstoff- und/oder Schwefelatom enthaltender aromatischer Kohlenwasserstoffrest mit 5 bis 15 Ringatomen je Rest ist,
- a: 1, 2 oder 3,
- b: 0, 1 oder 2,
- c: 0, 1 oder 2 sind,
- D' ,: E und F
jeweils Substituenten von C sind, wobei
- D': ein Rest der Formel

-(O)x-(R³)y-OH

ist, wobei
- x: 0 oder 1,
- y: 0 oder 1 sind,
- R³: ein linearer oder verzweigter, zweiwertiger Kohlenwasserstoffrest mit 1 bis 40 Kohlenstoffatomen je Rest, der gegebenenfalls durch mindestens ein Sauerstoffatom und/oder ein Schwefelatom und/oder eine Carboxylgruppe unterbrochen sein kann, ist,
- E: ein Rest der Formel

-O-R⁵
- F: ein Rest der Formel

-R⁶
- R²: ein Rest der Formel 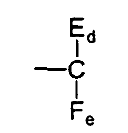 sind, wobei
- R⁵: ein einwertiger Kohlenwasserstoffrest mit 1 bis 18 Kohlenstoffatomen je Rest ist, der gegebenenfalls durch mindestens ein Sauerstoffatom unterbrochen sein kann,
- R⁶: ein einwertiger Kohlenwasserstoffrest mit 1 bis 18 Kohlenstoffatomen je Rest ist, der gegebenenfalls durch mindestens ein Sauerstoffatom unterbrochen sein kann,
- d: 0, 1 oder 2 und
- e: 0, 1 oder 2 sind,
mit einem Isocyanatgruppen enthaltenden Reagenz umgesetzt wird.

Bevorzugt werden als Hydroxylgruppen enthaltende Iodoniumsalze solche der allgemeinen Formel (X) eingesetzt, 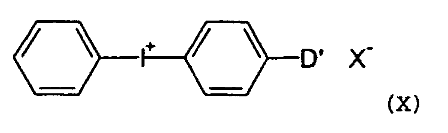 wobei D' und X⁻ die bereits angegebene Bedeutung haben.

Besonders bevorzugte Ausgangsmaterialien sind Iodoniumsalze der nachfolgenden Formel (XI):

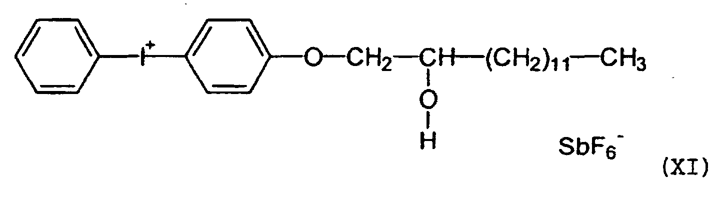

Beispiele für Isocyanatgruppen enthaltende Reagenzien, die mit Hydroxylgruppen tragenden Iodoniumsalzen umgesetzt werden können, sind solche mit der allgemeinen Formel (XII)

OCN-R⁴ (XII)

wobei
- R⁴: ein einwertiger linearer, verzweigter und/oder cyclischer Alkyl-, Aryl-, Haloalkyl- und/oder Haloarylradikalrest mit 1 bis 40 Kohlenstoffatomen ist, der gegebenenfalls durch mindestens ein Sauerstoffatom und/oder mindestens ein Schwefelatom und/oder mindestens eine Urethan- und/oder Estergruppe unterbrochen sein kann, und der gegebenenfalls hydrophobierende Substituenten und/oder mindestens eine Isocyanatgruppe enthalten kann.

Besonders bevorzugte Isocyanatgruppen enthaltende Reagenzien, die mit Hydroxylgruppen tragenden Iodoniumsalzen umgesetzt werden können, sind:
Alkylisocyanate, wie Methyl-, Ethyl-, Propyl-, Butyl-, sec. Butyl-, Cyclohexyl- Hexyl-, Octyl-, tert. Octyl-, Decyl-, Dodecyl-, und/oder Octadecylisocyanat;
Arylisocyanate, wie Phenyl-, Tolyl-, Dimethylphenyl-, Phenylethyl-, Propylphenyl-, Methoxyphenyl-, (Heptyloxy)phenyl-, Phenoxyphenyl-, Acetylphenyl-, Nitrophenyl-, Benzyl-, Methylbenzyl-, Methoxybenzyl-, Dimethyl-m-isopropenylbenzyl-, Naphtyl-, (Trifluormethyl)phenyl-, Di(trifluormethyl)phenyl-(Trifluormethylthio)phenyl- und/oder (Trifluormethoxy)-phenylisocyanat;
Haloalkylisocyanate, wie 2-Brommethylisocyanat; Haloarylisocyanate, wie Chlorbenzyl-, Chlorphenyl-, Trichlorphenyl-, 4-Brom-2,6-dimethylphenyl-, Fluorphenyl-, Difluorphenyl-, Fluoro-(trifluormethyl)phenyl- und/oder Fluorbenzylisocyanat;
Diisocyanate, wie Cyclohexan-, Dicyclohexylmethan-4,4'-, Diphenylmethan-4,4'-, Hexamethylen-1,6-, Isophoron-, und/oder Toluylendiisocyanat;
Polymere Isocyanate, wie Desomdur N 3300 von der Bayer AG, Desomdur L von der Bayer AG, Desmodur E41 von der Bayer AG, Desmodur Z4370 von der Bayer AG;
Polydimethylsiloxanisocyanate, wie

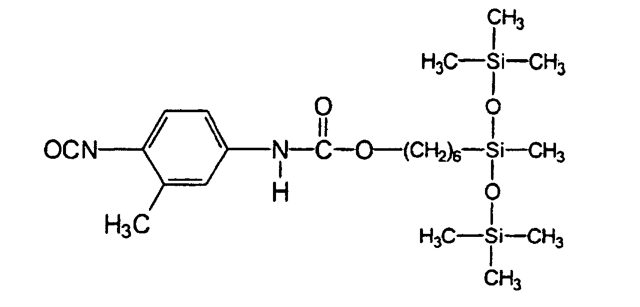

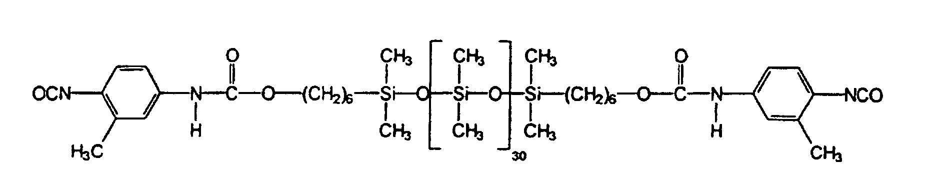

Die erfindungsgemäßen Iodoniumsalze eignen sich zum Beispiel als Photoinitiatoren für die Polymerisation von kationisch polymerisierbaren organischen Substanzen, wie Epoxiden, Vinylethern, Epoxygruppen enthaltenden Organopolysiloxanen, Alkenyloxygruppen, wie Vinyloxygruppen oder Propenyloxygruppen, enthaltenden Organopolysiloxanen und Olefinen. Solche Substanzen sind beispielsweise in US-A-5 057 549, DE-A-40 02 922 sowie in den eingangs genannten Patentschriften beschrieben.

### Ausführungsbeispiele:

### Beispiel 1

10 g eines handelsüblichen Photoinitiators gemäß der Formel (XI) werden in 10 ml Essigsäureethylester suspendiert, mit 1,0 g Ethylisocyanat und zwei Tropfen COSMOS® 29* als Katalysator gemischt und bei 60 °C im Wasserbad erwärmt. Das Gemisch wird 2 h gerührt. Nach der Reaktion wird das Lösemittel destillativ entfernt. Zurück bleibt ein gelbes, viskoses Produkt.
* Handelsprodukt der Goldschmidt AG

### Beispiel 2

10 g eines handelsüblichen Photoinitiators gemäß der Formel (XI) werden in 10 ml Essigsäureethylester suspendiert, mit 1,7 g Hexylisocyanat und zwei Tropfen COSMOS® 29 als Katalysator gemischt und bei 60 °C im Wasserbad erwärmt. Das Gemisch wird 2 h gerührt. Nach der Reaktion wird das Lösemittel destillativ entfernt. Zurück bleibt ein gelbes, viskoses Produkt.

### Beispiel 3

10 g eines handelsüblichen Photoinitiators gemäß der Formel (XI) werden in 10 ml Essigsäureethylester suspendiert, mit 1,6 g Phenylisocyanat und zwei Tropfen COSMOS® 29 als Katalysator gemischt und bei 60 °C im Wasserbad erwärmt. Das Gemisch wird 2 h gerührt. Nach der Reaktion wird das Lösemittel destillativ entfernt. Zurück bleibt ein gelbes, viskoses Produkt.

### Beispiel 4

10 g eines handelsüblichen Photoinitiators gemäß der Formel (XI) werden in 10 ml Essigsäureethylester suspendiert, mit 2,5 g m-(Trifluormethyl)phenylisocyanat und zwei Tropfen COSMOS® 29 als Katalysator gemischt und bei 60 °C im Wasserbad erwärmt. Das Gemisch wird 2 h gerührt. Nach der Reaktion wird das Lösemittel destillativ entfernt. Zurück bleibt ein gelbes, viskoses Produkt.

### Vergleich 1

Die Löslichkeit der erfindungsgemäßen Iodoniumsalze wird mit der Löslichkeit der Hydroxylgruppen tragenden Iodoniumsalze verglichen. Die Ergebnisse sind in Tabelle 1 zusammengefaßt:

**Tabelle 1**

| Photoinitiator | Löslichkeit in Toluol |
|---|---|
| Formel (XI) | unlöslich |
| Bsp. 1 | klar löslich |
| Bsp. 2 | klar löslich |
| Bsp. 3 | klar löslich |
| Bsp. 4 | klar löslich |

Der Löslichkeitsunterschied zwischen dem Hydroxylgruppen enthaltenden Photoinitiator mit der Formel (XI) und den erfindungsgemäßen, Urethangruppen enthaltenden Photoinitiatoren zur kationischen Härtung ist beträchtlich.

### Vergleich 2

Die Verminderung der Kristallisationsneigung läßt sich besonders deutlich anhand des Schmelzpunktes der Verbindungen aufzeigen.

**Tabelle 2**

| Photoinitiator | Schmelzpunkt |
|---|---|
| Formel (XI) | 91 °C |
| Bsp. 1 | viskoses Öl |
| Bsp. 2 | viskoses Öl |
| Bsp. 3 | viskoses Öl |
| Bsp. 4 | viskoses Öl |

Es zeigt sich, daß durch die Modifikation der Schmelzpunkt und damit die Kristallisationsneigung erheblich herabgesetzt wurde.

### Vergleich 3

Die verbesserte hydrolytische Stabilität der erfindungsgemäßen, Urethangruppen enthaltenden Iodoniumsalze läßt sich am besten im Vergleich mit dem Si-O-C-Bindungen enthaltenden Iodoniumsalz mit der Formel (XVI) (siehe Deutsche Patentanmeldung 19901531.7) zeigen.

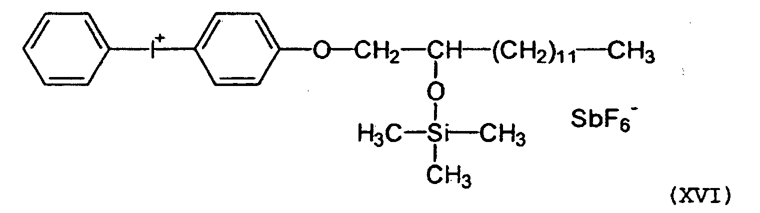

Die Ergebnisse sind in Tabelle 3 zusammengefaßt. Angegeben ist die Zeit bis zur Bildung sichtbarer Niederschläge bzw. Kristalle des hydrolytischen Spaltprodukts mit der Formel (XI):

| Photoinitiator | Stabilität in Toluol (50% w/w) | Stabilität in 1-Butanol (50% w/w) |
|---|---|---|
| Formel (XVI) | 21d | 2d |
| Bsp. 1 | >90d | >90d |
| Bsp. 2 | >90d | >90d |
| Bsp. 3 | >90d | >90d |
| Bsp. 4 | >90d | >90d |

### Versuche zur kationischen Photopolymerisation

Um die Aktivität, der in den Beispielen 1 - 4 dargestellten Verbindungen, in der kationischen Photopolymerisation zu überprüfen, wurden 98 Teile eines cycloaliphatischen Epoxysiloxans mit einem Epoxywert von 3,5 % und einer Viskosität von 125 mPas mit jeweils 2 Teilen Photoinitiator gemischt.

Die Mischungen wurden dann mit einer Technikumsbeschichtungsmaschine, ausgerüstet mit einem Fünf-Walzen-Antragswerk, auf eine Standard-OPP Folie (30µm) aufgetragen. Das Auftragsgewicht betrug 0,5 - 1 g/m². Die Beschichtungen wurden anschließend mit einer mikrowellenangeregten UV-Lampe (Fusion, 120 W/cm) bei einer Bahngeschwindigkeit von 20 m/min ausgehärtet.

Direkt nach dem Passieren der UV-Lampe wurde bestimmt, ob die Beschichtungen zu einem nicht mehr klebrigen Film ausgehärtet sind.

Die Ergebnisse sind in der folgenden Tabelle zusammengefaßt:

| Photoinitiator | Aussehen der Mischung | Aushärtung nach der UV-Lampe |
|---|---|---|
| Formel (VIII) | trübe, kristalliner Niederschlag | ungenügend; trüber, klebriger Film; Kristalle sichtbar |
| Bsp. 1 | opak | Aushärtung; nicht mehr klebriger Film |
| Bsp. 2 | klar | gute Aushärtung; klarer, nicht mehr klebriger Film |
| Bsp. 3 | klar | gute Aushärtung; klarer, nicht mehr klebriger Film |
| Bsp. 4 | klar | gute Aushärtung; klarer, nicht mehr klebriger Film |

Es zeigt sich, daß die Unterschiede im Härtungsverhalten zwischen dem Hydroxylgruppen enthaltenden Photoinitiator mit der Formel (VIII) und den erfindungsgemäßen, Urethangruppen enthaltenden Photoinitiatoren beträchtlich sind.

## Patentansprüche

1. Iodoniumsalze der allgemeinen Formel (IV)
[R¹-I-R²]⁺ X⁻ (IV)
wobei
I Iod ist,
X⁻ ein Anion eines komplexen Metallsalzes und/oder einer starken Säure ist,
R¹ der Rest 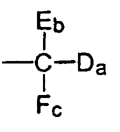 ist, wobei
C ein einwertiger aromatischer Kohlenwasserstoffrest mit 6 bis 14 Kohlenstoffatomen je Rest oder ein einwertiger, mindestens ein Sauerstoff- und/oder Schwefelatom enthaltender aromatischer Kohlenwasserstoffrest mit 5 bis 15 Ringatomen je Rest ist,
a 1, 2 oder 3,
b 0, 1 oder 2,
c 0, 1 oder 2 sind,
D, E und F jeweils Substituenten von C sind, wobei
D ein Rest der Formel 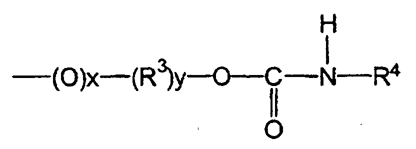 ist, wobei
x 0 oder 1,
y 0 oder 1 sind,
R³ ein linearer oder verzweigter, zweiwertiger Kohlenwasserstoffrest mit 1 bis 40 Kohlenstoffatomen je Rest, der gegebenenfalls durch mindestens ein Sauerstoffatom und/oder ein Schwefelatom und/oder eine Carboxylgruppe unterbrochen sein kann, ist,
R⁴ ein einwertiger linearer, verzweigter und/oder cyclischer Alkyl-, Aryl-, Haloalkyl- und/oder Haloarylradikalrest mit 1 bis 40 Kohlenstoffatomen ist, der gegebenenfalls durch mindestens ein Sauerstoffatom und/oder mindestens ein Schwefelatom und/oder mindestens eine Urethan- und/oder Estergruppe unterbrochen sein kann, und der gegebenenfalls hydrophobierende Substituenten und/oder mindestens eine Isocyanatgruppe enthalten kann,
E ein Rest der Formel
-O-R⁵
F ein Rest der Formel
-R⁶
R² ein Rest der Formel 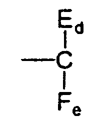
ist, wobei
R⁵ ein einwertiger Kohlenwasserstoffrest mit 1 bis 18 Kohlenstoffatomen je Rest ist, der gegebenenfalls durch mindestens ein Sauerstoffatom unterbrochen sein kann,
R⁶ ein einwertiger Kohlenwasserstoffrest mit 1 bis 18 Kohlenstoffatomen je Rest ist, der gegebenenfalls durch mindestens ein Sauerstoffatom unterbrochen sein kann,
d 0, 1 oder 2 und
e 0, 1 oder 2 sind.

2. Iodoniumsalze nach Anspruch 1, wobei mindestens einer der Reste R⁴, R⁵ und R⁶ ein Alkylrest, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest; ein Hexylrest, wie der n-Hexylrest, wie der Cyclohexylrest; ein Heptylrest, wie der n-Heptylrest; ein Octylrest, wie der n-Octylrest oder ein iso-Octylrest, wie der 2,2,4-Trimethylpentylrest; ein Nonylrest, wie der n-Nonylrest; ein Decylrest, wie der n-Decylrest; ein Dodecylrest, wie der n-Dodecylrest oder ein Octyldecylrest, wie der n-Octadecylrest ist.

3. Iodoniumsalze nach Anspruch 1, wobei mindestens einer der Reste R⁴, R⁵ und R⁶ ein Arylrest, wie der Phenyl-, Naphthyloder Anthrylrest ist.

4. Iodoniumsalze nach Anspruch 1, wobei mindestens einer der Reste R⁴, R⁵ und R⁶ ein Haloalkylrest, wie der Chlormethyl-, Trichlormethyl-, Trifluormethyl-, Pentafluorethyl- oder Heptafluorpropylrest ist.

5. Iodoniumsalze nach Anspruch 1, wobei mindestens einer der Reste R⁴, R⁵ und R⁶ ein Haloarylrest, wie der Bromphenyl-, Chlorphenyl-, Fluorphenyl-, Difluorphenyl-, Fluor(trifluormethyl)phenyl-, Pentafluorphenyl-, (Trifluormethyl)phenyl-, (Trifluormethylthio)phenyl- oder (Trifluormethoxy)phenylrest ist.

6. Iodoniumsalze nach Anspruch 1, wobei mindestens einer der Reste R⁴, R⁵ und R⁶ ein durch mindestens ein Sauerstoffatom und/oder Schwefelatom unterbrochener Kohlenwasserstoffrest der allgemeinen Formel -CH₂-CH₂-O-CH₃, -CH₂-CH₂O-CH₂CH₃ oder -CH₂-CH₂-S-CH₂CH₃ ist.

7. Iodoniumsalze nach Anspruch 1, wobei X⁻ unabhängig ausgewählt ist aus der Gruppe von Tosylat, SbF₆⁻, PF₆⁻, BF₄⁻, F₃CSO₃⁻, F₃CCO₂⁻, AsF₆⁻, ClO₄⁻, HSO₄⁻.

8. Iodoniumsalze nach Anspruch 1 mit der allgemeinen Formel (V) 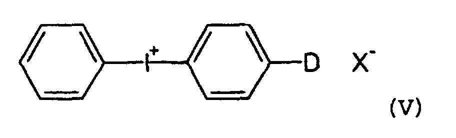 wobei D und X⁻ die bereits angegebene Bedeutung haben.

9. Verfahren zur Herstellung der Iodoniumsalze nach den Ansprüchen 1-8, **dadurch gekennzeichnet, daß** ein Hydroxylgruppen enthaltendes Iodoniumsalz mit der allgemeinen Formel (IX)
[R¹-I-R²]⁺ X⁻ (IX)
wobei
I Iod ist,
X⁻ ein Anion eines komplexen Metallsalzes und/oder einer starken Säure ist,
R¹ ein Rest 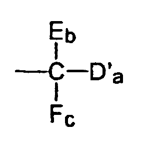
ist, worin
C ein einwertiger aromatischer Kohlenwasserstoffrest mit 6 bis 14 Kohlenstoffatomen je Rest oder ein einwertiger, mindestens ein Sauerstoff- und/oder Schwefelatom enthaltender aromatischer Kohlenwasserstoffrest mit 5 bis 15 Ringatomen je Rest ist,
a 1, 2 oder 3,
b 0, 1 oder 2,
c 0, 1 oder 2 sind,
D', E und F jeweils Substituenten von C sind, wobei
D' ein Rest der Formel
-(O)x-(R³)y-OH
ist, wobei
x 0 oder 1,
y 0 oder 1 sind,
R³ ein linearer oder verzweigter, zweiwertiger Kohlenwasserstoffrest mit 1 bis 40 Kohlenstoffatomen je Rest, der gegebenenfalls durch mindestens ein Sauerstoffatom und/oder ein Schwefelatom und/oder eine Carboxylgruppe unterbrochen sein kann, ist,
E ein Rest der Formel
-O-R⁵
F ein Rest der Formel
-R⁶
R² ein Rest der Formel 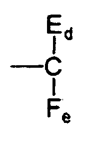
sind, wobei
R⁵ ein einwertiger Kohlenwasserstoffrest mit 1 bis 18 Kohlenstoffatomen je Rest ist, der gegebenenfalls durch mindestens ein Sauerstoffatom unterbrochen sein kann,
R⁶ ein einwertiger Kohlenwasserstoffrest mit 1 bis 18 Kohlenstoffatomen je Rest ist, der gegebenenfalls durch mindestens ein Sauerstoffatom unterbrochen sein kann,
d 0, 1 oder 2 und
e 0, 1 oder 2 sind,
mit einem Isocyanatgruppen enthaltenden Reagenz umgesetzt wird.

10. Verfahren nach Anspruch 9, **gekennzeichnet dadurch, daß** als Hydroxylgruppen enthaltende Iodoniumsalze solche der allgemeinen Formel (X) eingesetzt werden, 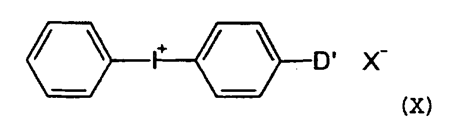 wobei D' und X⁻ die angegebene Bedeutung haben.

11. Verfahren nach Anspruch 9, **gekennzeichnet dadurch, daß** als Hydroxylgruppen enthaltende Iodoniumsalze solche der allgemeinen Formel (XI): 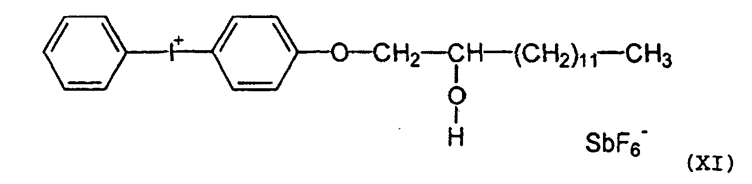 eingesetzt werden.

12. Verfahren nach Anspruch 9, **gekennzeichnet dadurch, daß** Isocyanatgruppen enthaltende Reagenzien der allgemeinen Formel (XII)
OCN-R⁴ (XII)
wobei
R⁴ ein einwertiger linearer, verzweigter und/oder cyclischer Alkyl-, Aryl-, Haloalkyl- und/oder Haloarylradikalrest mit 1 bis 40 Kohlenstoffatomen ist, der gegebenenfalls durch mindestens ein Sauerstoffatom und/oder mindestens ein Schwefelatom und/oder mindestens eine Urethan- und/oder Estergruppe unterbrochen sein kann, und der gegebenenfalls hydrophobierende Substituenten und/oder mindestens eine Isocyanatgruppe enthalten kann,
umgesetzt werden.

13. Verwendung der Iodoniumsalze nach den Ansprüchen 1-8 als Photoinitiatoren zur Polymerisation von kationisch polymerisierbaren Substanzen, insbesondere Epoxiden, Vinylethern, Epoxygruppen enthaltenden Organopolysiloxanen, Alkenyloxygruppen enthaltenden Organopolysiloxanen und/oder Olefinen.

## Claims

1. Iodonium salts of the general formula (IV)
[R¹-I-R²]⁺ X⁻ (IV)
where
I is iodine,.
X⁻ is the anion of a complex metal salt and/or of a strong acid,
R¹ is the radical 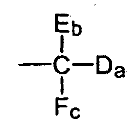 in which
C is a monovalent aromatic hydrocarbon radical. having 6 to 14 carbon atoms per radical or is a monovalent aromatic hydrocarbon radical containing at least one oxygen and/or sulphur atom and having 5 to 15 ring atoms per radical,
a is 1, 2 or 3,
b is 0, 1 or 2,
c is 0, 1 or 2,
D, E and F are each substituents of C,
D being a radical of the formula 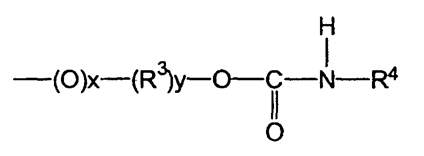 where
x is 0 or 1,
y is 0 or 1,
R³ is a linear or branched divalent hydrocarbon radical having 1 to 40 carbon atoms per radical, which can be interrupted if desired by at least one oxygen atom and/or one sulphur atom and/or one carboxyl group,
R⁴ is a monovalent linear, branched and/or cyclic alkyl, aryl, haloalkyl and/or haloaryl radical having 1 to 40 carbon atoms, which can be interrupted if desired by at least one oxygen atom and/or at least one sulphur atom and/or at least one urethane group and/or ester group, and which can if desired contain hydrophobicizing substituents and/or at least one isocyanate group, E is a radical of the formula
-O-R⁵
F is a radical of the formula
-R⁶
R² is a radical of the formula 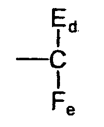 where
R⁵ is a monovalent hydrocarbon radical having 1 to 18 carbon atoms per radical, which can be interrupted if desired by at least one oxygen atom,
R⁶ is a monovalent hydrocarbon radical having 1 to 18 carbon atoms per radical, which can be interrupted if desired by at least one oxygen atom,
d is 0, 1 or 2, and
e is 0, 1 or 2.

2. Iodonium salts according to Claim 1, wherein at least one of the radicals R⁴, R⁵ and R⁶ is an alkyl radical, such as the methyl, ethyl, n-propyl, isopropyl, n-butyl, isobutyl, tert-butyl, n-pentyl, isopentyl, neopentyl, and tert-pentyl radical; a hexyl radical, such as the n-hexyl radical, for example, the cyclohexyl radical; a heptyl radical, such as the n-heptyl radical; an octyl radical, such as the n-octyl radical or an isooctyl radical, such as the 2,2,4-trimethylpentyl radical; a nonyl radical, such as the n-nonyl radical; a decyl radical, such as the n-decyl radical; a dodecyl radical, such as the n-dodecyl radical, or an octadecyl radical, such as the n-octadecyl radical.

3. Iodonium salts according to Claim 1, wherein at least one of the radicals R⁴, R⁵ and R⁶ is an aryl radical, such as the phenyl, naphthyl or anthryl radical.

4. Iodonium salts according to Claim 1, wherein at least one of the radicals R⁴, R⁵ and R⁶ is a haloalkyl radical, such as the chloromethyl, trichloromethyl, trifluoromethyl, pentafluoroethyl or heptafluoropropyl radical.

5. Iodonium salts according to Claim 1, wherein at least one of the radicals R⁴, R⁵ and R⁶ is a haloaryl radical, such as the bromophenyl, chlorophenyl, fluorophenyl, difluorophenyl, fluoro(trifluoromethyl)phenyl, pentafluorophenyl, (trifluoromethyl)phenyl, (trifluoromethylthio)phenyl or (trifluoromethoxy)phenyl radical.

6. Iodonium salts according to Claim 1, wherein at least one of the radicals R⁴, R⁵ and R⁶ is a hydrocarbon radical, interrupted by at least one oxygen atom and/or sulphur atom, of the general formula -CH₂-CH₂-O-CH₃, -CH₂-CH₂O-CH₂CH₃ or -CH₂-CH₂-S-CH₂CH₃.

7. Iodonium salts according to Claim 1, wherein X⁻ is selected independently from the group consisting of tosylate, SbF₆⁻, PF₆⁻, BF₄⁻, F₃CSO₃⁻, F₃CCO₂⁻, AsF₆⁻, ClO₄⁻, and HSO₄⁻.

8. Iodonium salts according to Claim 1 with the general formula (V) 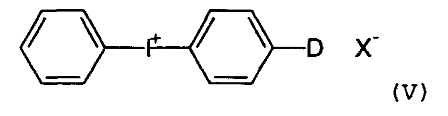 wherein D and X⁻ are as already defined.

9. Process for preparing the iodonium salts according to Claims 1-8, **characterized in that** it comprises reacting a hydroxyl-containing iodonium salt having the general formula (IX)
[R¹-I-R²]⁺ X⁻ (IX)
in which
I is iodine,
X⁻ is the anion of a complex metal salt and/or of a strong acid,
R¹ is a radical 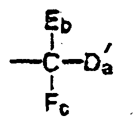 in which
C is a monovalent aromatic hydrocarbon radical having 6 to 14 carbon atoms per radical or is a monovalent aromatic hydrocarbon radical containing at least one oxygen and/or sulphur atom and having 5 to 15 ring atoms per radical,
a is 1, 2 or 3,
b is 0, 1 or 2,
c is 0, 1 or 2,
D', E and F are each substituents of C,
D' being a radical of the formula
**-(O)x-(R**^{**3**}**)y-OH**
where
x is 0 or 1,
y is 0 or 1,
R³ is a linear or branched divalent hydrocarbon radical having 1 to 40 carbon atoms per radical, which can be interrupted if desired by at least one oxygen atom and/or one sulphur atom and/or one carboxyl group,
E is a radical of the formula
-O-R⁵
F is a radical of the formula
-R⁶
R² is a radical of the formula 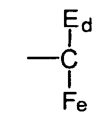 where
R⁵ is a monovalent hydrocarbon radical having 1 to 18 carbon atoms per radical, which can be interrupted if desired by at least one oxygen atom,
R⁶ is a monovalent hydrocarbon radical having 1 to 18 carbon atoms per radical, which can be interrupted if desired by at least one oxygen atom,
d is 0, 1 or 2, and
e is 0, 1 or 2,
with a reagent containing isocyanate groups.

10. Process according to Claim 9, **characterized in that** hydroxyl-containing iodonium salts used are those of the general formula (X) 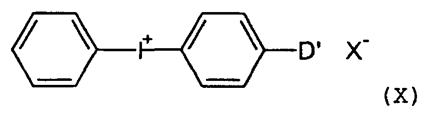 wherein D' and X⁻ are as defined.

11. Process according to Claim 9, **characterized in that** hydroxyl-containing iodonium salts used are those of the general formula (XI):

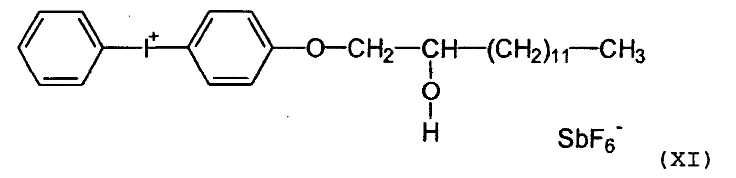

12. Process according to Claim 9, **characterized in that** reagents containing isocyanate groups, of the general formula (XII)
OCN-R⁴ (XII),
are reacted, where
R⁴ is a monovalent linear, branched and/or cyclic alkyl, aryl, haloalkyl and/or haloaryl radical having 1 to 40 carbon atoms, which can if desired be interrupted by at least one oxygen atom and/or at least one sulphur atom and/or at least one urethane group and/or ester group, and which can if desired contain hydrophobicizing substituents and/or at least one isocyanate group.

13. Use of the iodonium salts according to Claims 1-8 as photoinitiators for polymerizing cationically polymerizable substances, especially epoxides, vinyl ethers, organopolysiloxanes containing epoxy groups, organopolysiloxanes containing alkenyloxy groups, and/or olefins.

## Revendications

1. Sels d'iodonium de formule générale (IV)
[R¹-I-R²]⁺ X⁻ (IV)
dans laquelle
I représente un atome d'iode,
X⁻ représente un anion d'un sel métallique complexe et/ou d'un acide fort,
R¹ représente le radical 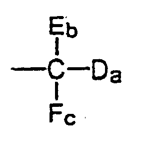 dans lequel
C représente un radical hydrocarboné aromatique monovalent comprenant 6 à 14 atomes de carbone par radical ou un radical hydrocarboné aromatique monovalent, contenant au moins un atome d'oxygène et/ou de soufre comprenant 5 à 15 atomes dans le cycle par radical,
a représente 1, 2 ou 3,
b représente 0, 1 ou 2,
c représente 0, 1 ou 2,
D, E et F représentent à chaque fois des substituants de C,
dans lequel
D représente un radical de formule 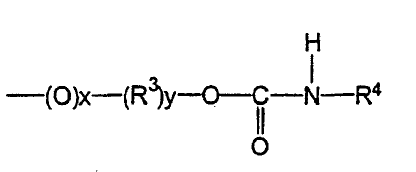 dans laquelle
x représente 0 ou 1,
y représente 0 ou 1,
R³ représente un radical hydrocarboné linéaire ou ramifié, divalent comprenant 1 à 40 atomes de carbone par radical, qui peut le cas échéant être interrompu par un atome d'oxygène et/ou de soufre et/ou un groupement carboxyle,
R⁴ représente un radical alkyle, aryle, halogénoalkyle et/ou halogénoaryle monovalent, linéaire, ramifié et/ou cyclique comprenant 1 à 40 atomes de carbone, qui peut le cas échéant être interrompu par au moins un atome d'oxygène et/ou au moins un atome de soufre et/ou au moins un groupement uréthane et/ou ester et qui peut le cas échéant contenir des substituants à effet hydrophobe et/ou au moins un groupement isocyanate,
E représente un radical de formule
-O-R⁵
F représente un radical de formule
-R⁶
R² représente un radical de formule 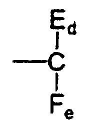 dans lesquelles
R⁵ représente un radical hydrocarboné monovalent comprenant 1 à 18 atomes de carbone par radical, qui peut le cas échéant être interrompu par au moins un atome d'oxygène,
R⁶ représente un radical hydrocarboné monovalent comprenant 1 à 18 atomes de carbone par radical, qui peut le cas échéant être interrompu par au moins un atome d'oxygène,
d représente 0, 1 ou 2 et
e représente 0, 1 ou 2.

2. Sels d'iodonium selon la revendication 1, dans lesquels au moins un des radicaux R⁴, R⁵ et R⁶ représente un radical alkyle, tel que le radical méthyle, éthyle, n-propyle, iso-propyle, n-butyle, iso-butyle, tert.-butyle, n-pentyle, iso-pentyle, néo-pentyle, tert.-pentyle ; un radical hexyle, tel que le radical n-hexyle, le radical cyclohexyle ; un radical heptyle, tel que le radical n-heptyle ; un radical octyle, tel que le radical n-octyle ou un radical iso-octyle, tel que le radical 2,2,4-triméthylpentyle ; un radical nonyle, tel que le radical n-nonyle ; un radical décyle, tel que le radical n-décyle ; un radical dodécyle, tel que le radical n-dodécyle ou un radical octyldécyle, tel que le radical n-octadécyle.

3. Sels d'iodonium selon la revendication 1, dans lesquels au moins un des radicaux R⁴, R⁵ et R⁶ représente un radical aryle, tel que le radical phényle, naphtyle ou anthryle.

4. Sels d'iodonium selon la revendication 1, dans lesquels au moins un des radicaux R⁴, R⁵ et R⁶ représente un radical halogénoalkyle, tel que le radical chlorométhyle, trichlorométhyle, trifluorométhyle, pentafluoroéthyle ou heptafluoropropyle.

5. Sels d'iodonium selon la revendication 1, dans lesquels au moins un des radicaux R⁴, R⁵ et R⁶ représente un radical halogénoaryle, tel que le radical bromophényle, chlorophényle, fluorophényle, difluorophényle, fluoro(tri-fluorométhyl)phényle, pentafluorophényle, (trifluorométhyl)phényle, (trifluorométhylthio)phényle ou (trifluorométhoxy)-phényle.

6. Sels d'iodonium selon la revendication 1, dans lesquels au moins un des radicaux R⁴, R⁵ et R⁶ représente un radical hydrocarboné interrompu par au moins un atome d'oxygène et/ou de soufre, de formule générale -CH₂-CH₂-O-CH₃, -CH₂-CH₂O-CH₂CH₃ ou -CH₂-CH₂-S-CH₂CH₃.

7. Sels d'iodonium selon la revendication 1, dans lesquels X⁻ est choisi indépendamment dans le groupe constitué de tosylate, SbF₆⁻, PF₆⁻, BF₄⁻, F₃CSO₃⁻, F₃CCO₂⁻, AsF₆⁻, ClO₄⁻, HSO₄⁻.

8. Sels d'iodonium selon la revendication 1 présentant la formule générale (V) 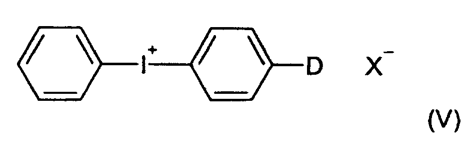 dans laquelle D et X⁻ ont les significations déjà indiquées.

9. Procédé pour la préparation des sels d'iodonium selon les revendications 1 à 8, **caractérisé en ce qu'**on transforme, avec un réactif contenant des groupements isocyanate, un sel d'iodonium contenant des groupements hydroxyle de formule générale (IX)
[R¹-I-R²]⁺ X⁻ (IX)
dans laquelle
I représente un atome d'iode,
X⁻ représente un anion d'un sel métallique complexe et/ou d'un acide fort,
R¹ représente un radical 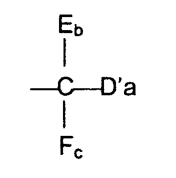 dans lequel
C représente un radical hydrocarboné aromatique monovalent comprenant 6 à 14 atomes de carbone par radical ou un radical hydrocarboné aromatique monovalent, contenant au moins un atome d'oxygène et/ou de soufre comprenant 5 à 15 atomes dans le cycle par radical,
a représente 1, 2 ou 3,
b représente 0, 1 ou 2,
c représente 0, 1 ou 2,
D', E et F représentent à chaque fois des substituants de C,
dans lequel
D' représente un radical de formule
-(O)ₓ-(R³)_{y}-OH
dans laquelle
x représente 0 ou 1,
y représente 0 ou 1,
R³ représente un radical hydrocarboné linéaire ou ramifié, divalent comprenant 1 à 40 atomes de carbone par radical, qui peut le cas échéant être interrompu par au moins un atome d'oxygène et/ou de soufre et/ou un groupement carboxyle,
E représente un radical de formule
-O-R⁵
F représente un radical de formule
-R⁶
R² représente un radical de formule 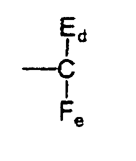 dans lesquelles
R⁵ représente un radical hydrocarboné monovalent comprenant 1 à 18 atomes de carbone par radical, qui peut le cas échéant être interrompu par au moins un atome d'oxygène,
R⁶ représente un radical hydrocarboné monovalent comprenant 1 à 18 atomes de carbone par radical, qui peut le cas échéant être interrompu par au moins un atome d'oxygène,
d représente 0, 1 ou 2 et
e représente 0, 1 ou 2.

10. Procédé selon la revendication 9, **caractérisé en ce qu'**on utilise comme sels d'iodonium contenant des groupements hydroxyle ceux de formule générale (X) 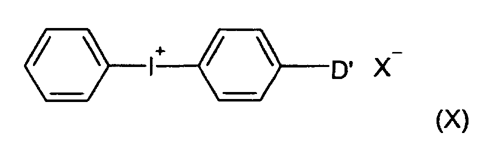 dans laquelle D' et X⁻ ont la signification indiquée.

11. Procédé selon la revendication 9, **caractérisé en ce qu'**on utilise comme sels d'iodonium contenant des groupements hydroxyle ceux de formule générale (XI)

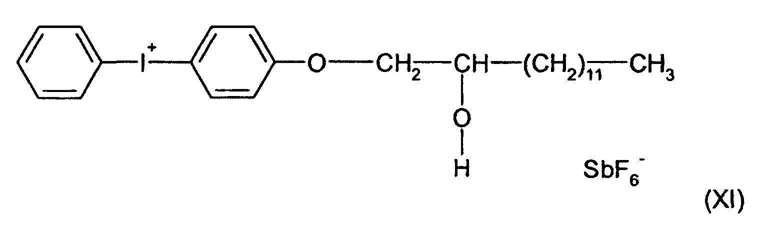

12. Procédé selon la revendication 9, **caractérisé en ce qu'**on transforme des réactifs contenant des groupements isocyanate de formule générale (XII)
OCN-R⁴ (XII)
dans laquelle
R⁴ représente un radical alkyle, aryle, halogénoalkyle
et/ou halogénoaryle monovalent, linéaire, ramifié et/ou cyclique comprenant 1 à 40 atomes de carbone, qui peut le cas échéant être interrompu par au moins un atome d'oxygène et/ou au moins un atome de soufre et/ou au moins un groupement uréthane et/ou ester et qui peut le cas échéant contenir des substituants à effet hydrophobe et/ou au moins un groupement isocyanate.

13. Utilisation des sels d'iodonium selon les revendications 1 à 8 comme photo-initiateurs pour la polymérisation de substances polymérisables par voie cationique, en particulier les époxydes, les vinyléthers, les organopolysiloxanes contenant des groupements époxy, les organopolysiloxanes contenant des groupements alcényloxy et/ou les oléfines.
